# EUROPEAN PATENT SPECIFICATION

(11) **EP 3 592 235 B1**
(45) Date of publication and mention of the grant of the patent: **08.12.2021**
(21) Application number: 18711638.9
(22) Date of filing: 08.03.2018
(51) Int. Cl.: A61B 6/06, G01T 1/24, G01T 1/29, A61B 5/06, A61N 5/10, A61B 6/00

(54) **RADIATION DETECTOR**
STRAHLUNGSDETEKTOR
DÉTECTEUR DE RADIATION

(30) Priority: 09.03.2017 GB 201703785
(43) Date of publication of application: 15.01.2020
(73) Proprietor: The University of Bristol, Bristol BS8 1QU (GB); Swansea University, Swansea SA2 8PP (GB); University Hospitals Bristol NHS Foundation Trust, Bristol BS1 3NU (GB)
(72) Inventor: VELTHUIS, Johannes Jarig, Bristol BS8 1TH (GB); PAGE, Ryan Frank, Bristol BS8 1TH (GB); HUGTENBURG, Richard Peter, Swansea SA2 8PP (GB); STEVENS, Paul, Bristol BS1 3NU (GB)
(74) Representative: Ribeiro, James Michael
(86) International application number: PCT/GB2018/050583
(87) International publication number: WO 2018/162904

(56) References cited:
- EP-A1- 2 733 507
- US-A- 3 775 646
- US-A- 5 017 989
- US-A- 5 198 673
- US-A1- 2006 049 362
- US-A1- 2010 133 441
- US-A1- 2010 133 584
- US-A1- 2011 079 728

## Description

### Technical Field

The present invention relates to a radiation detector, typically but not exclusively an upstream detector for use in radiotherapy treatment.

### Background to the Invention

Many cancer patients are treated using high energy X-ray photon irradiation. For some cancers this presents the difficulty that the tumour might be located very close to healthy body tissue that is radiation-sensitive. A recent development in ionizing radiation therapy is to produce a strongly position-dependent intensity variation within a single beam by combining many intricate beam shapes which can, for example, be created by thin tungsten leaf collimators absorbing discrete regions of an X-ray beam. The leaf motion may either be in discrete steps or continuous. In a technique often called Intensity Modulated Radiation Therapy (IMRT), typically about seven such beams are combined from different static directions by means of a rotating gantry. Alternatively, both the leaf positions and the beam direction are continuously updated while the beam is on - a technique often referred to as Volumetric Arc Therapy (VMAT).

Before a patient is treated with such a complicated beam configuration, a pre-treatment measurement needs to be made. In this measurement, the treatment program is executed and the dose is measured using a water phantom. The need for the pre-treatment measurement therefore increases the overall time required to treat a patient.

A more desirable method of measuring the radiation dose given to a patient is to carry out the measurement during the treatment itself, but this can be challenging. Measuring the dose using a detector positioned downstream of the patient is notoriously difficult as the patient acts as a very complicated scattering centre.

For this reason a detector positioned upstream of the patient is preferable. However, upstream detectors attenuate the radiation beam and change the energy spectrum of the beam. Monolithic Active Pixel Sensors (MAPS) are ideal detectors to measure the 2D profile of a radiotherapy beam upstream. MAPS can be made very thin, so as to reduce attenuation, whilst still having a good signal-to-noise performance. One such detector is described in International patent no. WO2012/085507. However, a remaining challenge with thin, upstream sensors is that the detectors are sensitive to the signals of both the therapeutic photons and unwanted background electron and positron contamination, making the dose measurement difficult to extract.

Accordingly, there is a need for an upstream detector for use in radiotherapy which enables the dose delivered by the radiation beam to be more readily isolated and accurately measured.

US2011079728 discloses a detector having an entrance opening etched through a low-resistivity volume of silicon, a sensitive volume of high-resistivity silicon for converting the radiation particles into detectable charges, and a passivation layer between the low and high-resistivity silicon layers. The detector also has electrodes built in the form of vertical channels for collecting the charges generated in the sensitive volume, and read-out electronics for generating signals based on the collected charges.

US2010133584 discloses a semiconductor device structure comprising a first bulk crystal semiconductor material and a second bulk crystal semiconductor material provided on a surface of the first bulk crystal semiconductor material with or without a deliberate intermediate region.

### Summary of Invention

The invention is defined in independent claims 1, 14 and 15. Further aspects and preferred embodiments are defined in the appended claims. Aspects, embodiments and examples of the present disclosure which do not fall under the scope of the appended claims do not form part of the invention and are merely provided for illustrative purposes.

A first aspect of the invention provides a radiation detector according to claim 1.

A patterned structure is arranged to generate charged particles in response to photons which are incident on the patterned structure. These charged particles may be electrons, positrons, holes or any other charged particle. The patterned structure is patterned with a varying thickness so that it has a plurality of thick regions interspersed with a plurality of thin regions, a thickness of each of the thick regions being greater than a thickness of each of the thin regions. The patterned structure may be manufactured by removing material to form the thin regions, or by adding material to form the thick regions.

Typically each of the thick regions is a few times thicker than each of the thin regions. For instance an average thickness of the thick regions may be more than two, three or four times an average thickness of the thin regions.

A signal generation layer is arranged to generate signal charge in response to charged particles generated in the patterned structure which are incident on the signal generation layer. The signal generation layer may be a solid, liquid or gas.

Typically an average thickness of the thick regions is of about the same order as an average thickness of the signal generation layer - a ratio between the average thicknesses being no more than three, for example.

A barrier layer is arranged so that the signal generation layer is positioned between the patterned structure and barrier layer, and in contact with the patterned structure and barrier layer. The patterned structure and barrier layer trap the signal charge in the signal generation layer.

The patterned structure and barrier layer create barriers trapping the signal charge in the signal generation layer. Optionally the patterned structure, signal generation layer and barrier layer are semiconductor layers with different doping concentrations, and the different doping concentrations create the barriers trapping the signal charge in the signal generation layer.

A plurality of collectors are provided, each collector arranged to collect the signal charge trapped in the signal generation layer. Typically the collectors are in the barrier layer.

Typically the collectors comprise a first collector adjacent a first one of the thin regions and a second collector adjacent a first one of the thick regions.

Preferably the radiation detector is operated to obtain a first signal from the first collector; obtain a second signal from the second collector; and correct the second signal using the first signal to obtain an output originating exclusively from charged particles generated from photon interactions in the first one of the thick regions.

Typically a total number of the collectors is greater than a sum of the number of thick regions and the number of thin regions. Preferably the total number of collectors is more than four times greater, and most preferably it is more than ten times greater than the sum of the number of thick regions and the number of thin regions.

Typically the thin and thick regions of the patterned structure form a regular pattern, for instance elongated trenches and ridges, a chequered pattern or other repeating pattern. The thin and thick regions may together form a single layer, or they may be formed by different parts - for instance a thin layer carrying strips or a chequered pattern of pads.

Optionally the thickness of each of the thick regions is substantially constant across its entire area, and the thickness of each of the thin regions is substantially constant across its entire area. Alternatively the thicknesses may vary across their respective areas.

Optionally the thickness of the thick regions is the same for all of the thick regions, and the thickness of the thin regions is the same for all of the thin regions. Alternatively they may vary from region to region.

Preferably a maximum or average thickness of each of the thick regions of the patterned structure is less than 200*µ*m, and most preferably a maximum or average thickness of each of the thick regions of the patterned structure is less than 100*µ*m.

The thickness of each of the thin regions of the patterned structure is non-zero. The minimum or average thickness of each of the thin regions of the patterned structure may be greater than 1*µ*m for example.

Preferably a maximum or average thickness of each of the thin regions of the patterned structure is less than 20*µ*m, and most preferably a maximum or average thickness of each of the thin regions of the patterned structure is less than 10*µ*m.

A sum of the thicknesses of the patterned structure, the barrier layer and the signal generation layer has a maximum which is typically less than 500*µ*m, preferably less than 300*µ*m and most preferably less than 200*µ*m.

A total thickness of the radiation detector is typically less than 500*µ*m, preferably less than 300*µ*m and most preferably less than 200*µ*m.

Other preferred features of the detector are set out in the dependent claims.

A second aspect of the invention provides a radiation detection system comprising: the radiation detector of the first aspect, wherein the collectors comprise a first collector adjacent a first one of the thin regions and a second collector adjacent a first one of the thick regions; and an analysis unit coupled to the radiation detector, wherein the analysis unit is arranged to: obtain a first signal from the first collector; obtain a second signal from the second collector; and correct the second signal using the first signal to obtain an output. For instance the second signal can be corrected by subtracting the first signal from the second signal to obtain the output, dividing the second signal by the first signal, or any other suitable correction method. Typically the output generated by the correction of the second signal is a signal from the second collector originating exclusively from electrons or other charged particles generated in the thick region directly above it.

The radiation detection system may be a radiotherapy system comprising a radiation beam source for generate a radiation beam wherein the radiation detector is arranged to detect photons from the radiation beam. Alternatively the radiation detection system may be used for other purposes - for instance to monitor background radiation in a nuclear environment such as a nuclear laboratory, a nuclear power facility, or a nuclear storage facility.

A third aspect of the invention provides a radiotherapy system comprising: a radiation beam source for generate a radiation beam; and a radiation detector according to the first aspect of the invention arranged to detect photons from the radiation beam.

Other preferred features of the radiotherapy system detector are set out in the dependent claims.

An analysis unit is typically coupled to the radiation detector, the analysis unit arranged to receive signals from the radiation detector, and generate an output which can be used to control the radiation beam source. For instance the output may be used to control a multi-leaf collimator of the radiation beam source. Alternatively the output could be used for treatment verification and monitoring.

### Brief Description of the Drawings

Embodiments of the invention will now be described, strictly by way of example only, with reference to the accompanying drawings, of which:
Figure 1 is a schematic of a radiotherapy treatment system according to an embodiment of the present invention;
Figure 2 is a multileaf collimator for use in the system of Figure 1;
Figure 3 is a schematic cross-sectional view of a conventional MAPS detector;
Figure 4 is a schematic cross-sectional view showing a number of radiation-detector interaction processes;
Figure 5a is a schematic view of a radiation detector according to an embodiment of the present invention;
Figure 5b shows the in-pixel circuitry for a single pixel of the radiation detector;
Figure 5c shows an alternative radiation detector with a patterned structure formed by multiple parts;
Figure 6a is a schematic of a patterned sensor with the pattern taking the form of alternating trenches and ridges;
Figure 6b is a plan view of a patterned sensor with the pattern taking the form of a chequered pattern; and
Figure 7 is a perspective view of a detector patterned on two sides according to an embodiment of the present invention;
Figure 8 is a perspective view of a sensor and its housing according to an embodiment of the present invention;
Figure 9 is a perspective view of a 3D sensor according to an embodiment of the present invention.

### Description of the Embodiments

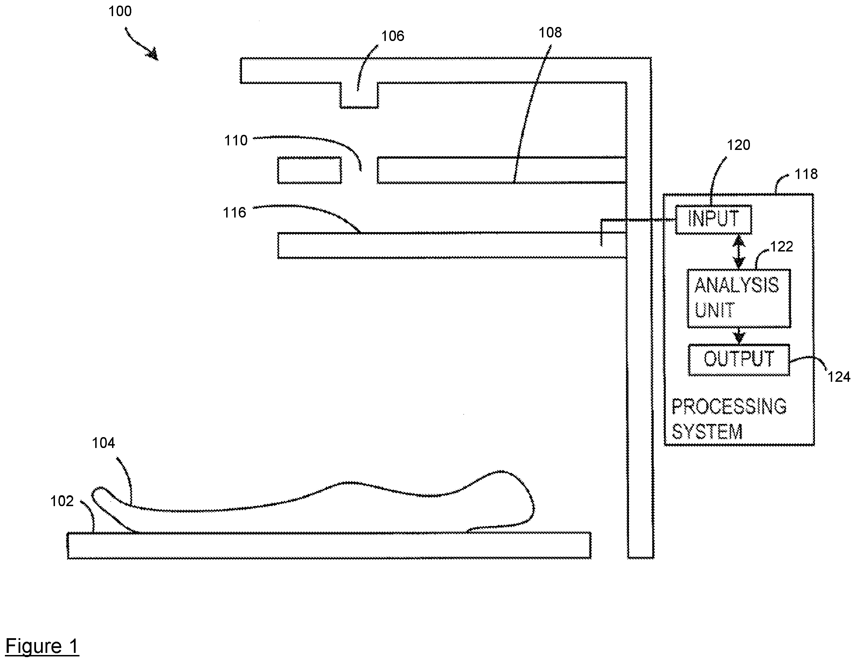

Referring first to Figure 1, a radiotherapy treatment system is shown generally at 100. The system comprises a surface 102 on which a patient 104 receiving the radiotherapy treatment can be positioned, and a radiation source 106 that is able to direct radiation towards the patient 104. The radiation source 106 may be a source of X-rays for example.

For example, the radiation source 106 can operate by accelerating electrons in a small linear accelerator. These electrons collide with a target, generating Bremsstrahlung X-rays. These X-rays have a varying energy. In addition, secondary electrons are generated. This results in a radiation beam comprising both secondary electrons and X-rays, although the dose delivered to a tumour inside the patient is solely due to the X-rays in the beam.

Located between the radiation source 106 and the patient 104 is a multi-leaf collimator 108, which defines an opening 110 through which radiation can pass.

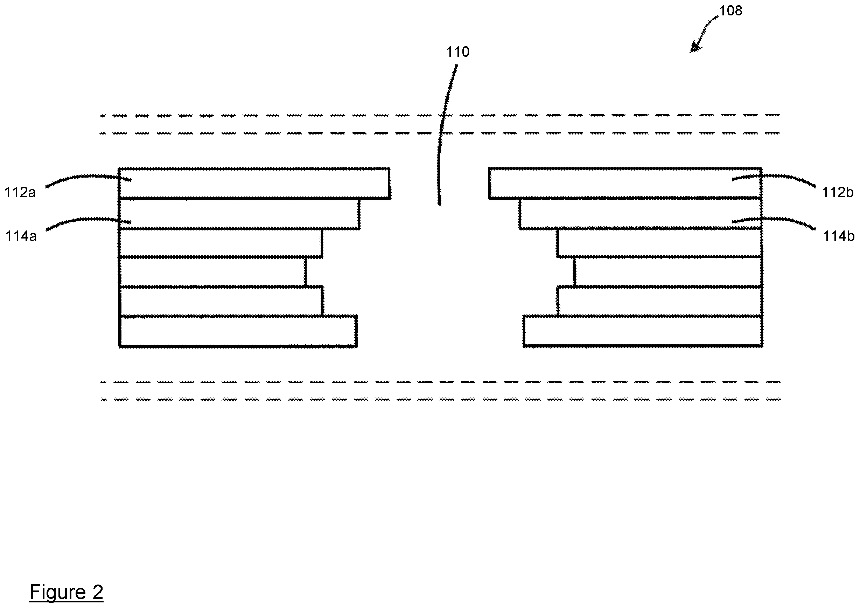

Referring now to Figure 2, which is a view of the collimator 108 from above, the collimator 108 includes multiple leaves 112a, 112b; 114a, 114b; etc. The leaves are made of a material, such as tungsten, which is opaque to the radiation. The leaves of each pair can be moved towards or away from each other, so as to define the shape of the opening 110 through which the radiation can pass.

Controlling the size and shape of the opening 110 through the movement of the collimator leaves 112a, 112b; 114a, 114b; etc. therefore results in a radiation beam with a highly controllable shape and intensity. This allows the radiation dose supplied to the patient 104 to be controlled.

Referring back to Figure 1, the gantry carrying the radiation source 106 can be rotated about an axis that might for example lie along the length of the patient support surface 102. In this case, both the beam direction and the leaf positions are continuously updated whilst the beam is switched on, in a technique known as Volumetric Arc Therapy (VMAT).

Located between the collimator 108 and the patient 104 is an upstream X-ray radiation detector 116. As an example, the distance between the sensor 116 and the collimator opening 110 may be about 10cm. The detector 116 may, for example, be a large area, radiation hard, Monolithic Active Pixel Sensor (MAPS).

The detector 116 is connected to a processing system 118, which includes an input 120 for receiving electronic signals generated by the detector 116, an analysis unit 122, and an output 124 for providing results to a user of the system, for example visually, either in real time or after the event, or by means of an audible alarm in the event that the detected beam is not as intended. The analysis unit 122 is able to detect a signal level for each pixel of the detector in each frame period. The analysis unit 122 is then able to generate an output that indicates a configuration of the X-ray beam based on the pattern of pixels in which there have been a certain number of hits over a particular time period. This output may then be used to reconfigure the shape of the collimator leaves 112a, 112b; 114a, 114b; etc. to achieve the desired configuration of the X-ray beam and/or register the deviation in order to apply a correction later on, possibly in a subsequent treatment. The analysis unit 122 may take the form of a suitably programmed computer.

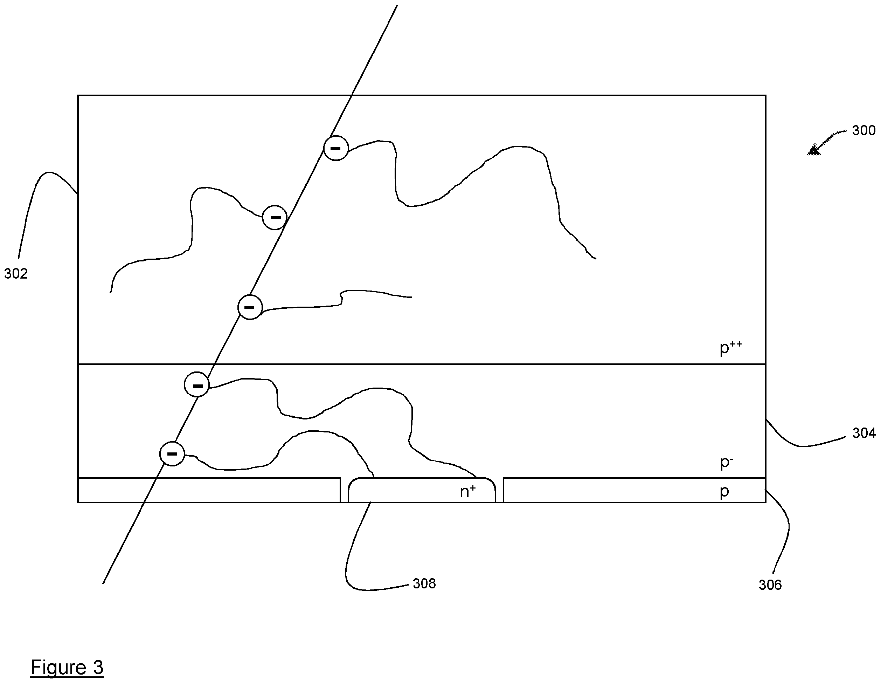

Figure 3 is a schematic cross-section through a conventional MAPS detector 300, provided purely for the purposes of illustration. The MAPS detector 300 comprises a sandwich structure including a highly doped (p⁺⁺) bulk layer 302. On the bulk layer 302 is grown a layer of lightly doped p-type material. This layer is known as the sensitive layer 304, and the required electronic circuitry is formed on this to provide an electronics layer 306. The electronics layer 306 is formed as a further layer on the sensitive layer 304, such that the sensitive layer 304 is sandwiched between the bulk layer 302 and the electronics layer 306. The electronics layer 306 comprises a layer of p-type material, throughout which a plurality of collection diodes 308 are positioned, along with transistors and other electronic circuitry (not shown).

The beam directed towards the patient comprises both therapeutic radiation and background electrons. Background electrons may comprise electrons from a number of sources, which may include the linear accelerator head, the air, and the housing of the detector. Signal generation in the MAPS detector is a result of the interactions of the radiation and background electrons with the MAPS detector.

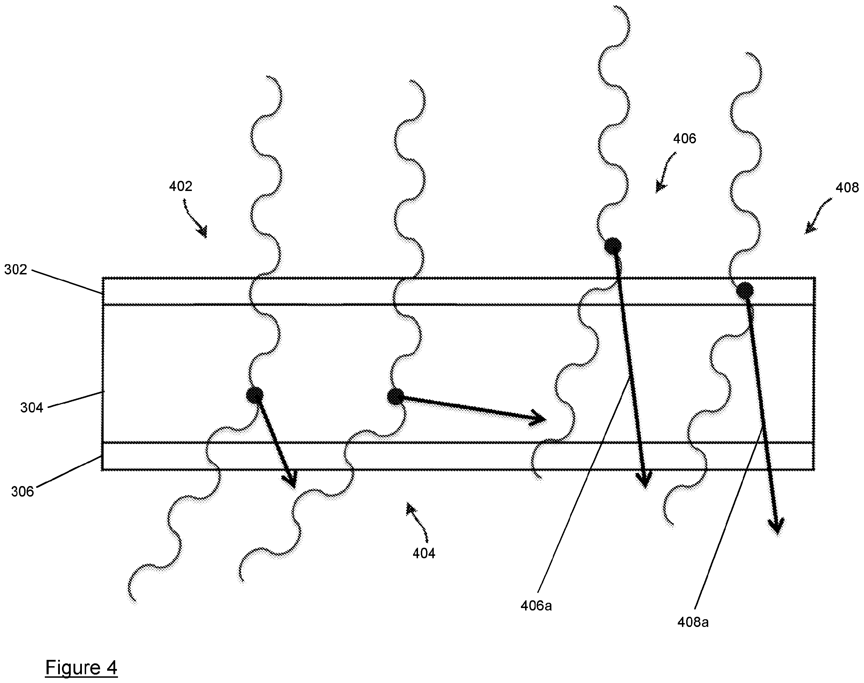

Interactions of the therapeutic photons with the conventional MAPS detector 300 of Figure 3 will now be described with reference to Figure 4.

X-ray photons or charged particles may interact with the sensitive layer 304 of the detector. This may result in the production of electron-hole pairs. The electrons or holes in the sensitive layer 304 will remain confined in the sensitive layer 304. These electrons or holes will therefore perform a random walk through the sensitive layer 304. If such an electron or hole gets close enough to a collection diode 308, the electron or hole is removed from the sensitive layer 304 and read out. Pixel sizes typically range from about 5 µm to 50 µm. The electrons or holes collected by the collection diode provide signal charge for the detector.

These processes in the sensitive layer 304 are illustrated by processes 402, 404 in Figure 4.

Electrons may also be generated before the photons reach the MAPS detector, due to interactions in the air or with the accelerator. This process is shown at 406 and generates electrons 406a.

Further still, electron-hole pairs may be generated in the bulk layer 302 in response to incident photons. This process is shown at 408 and generates electrons 408a (as well as holes, not shown).

The cross section for the processes described above is very low, meaning that most of the beam is transmitted undisturbed. However, the electrons 406a, 408a generated by processes 406 and 408 have a very high interaction cross section with the detector. So electron-hole pairs are generated in the sensitive layer 304 in response to electrons 406a, 408a from processes 406 and 408 which are incident on the sensitive layer 304. The energy deposited by these electrons 406a, 408a follows the well-known Landau distribution reasonably well, and generates a most probable signal of approximately 80 signal electron-hole pairs per micron in the sensitive layer 304. The electrons or holes generated in the sensitive layer 304 in response to the electrons 406a, 408a can then be collected by the collection diodes to provide further signal charge for the detector.

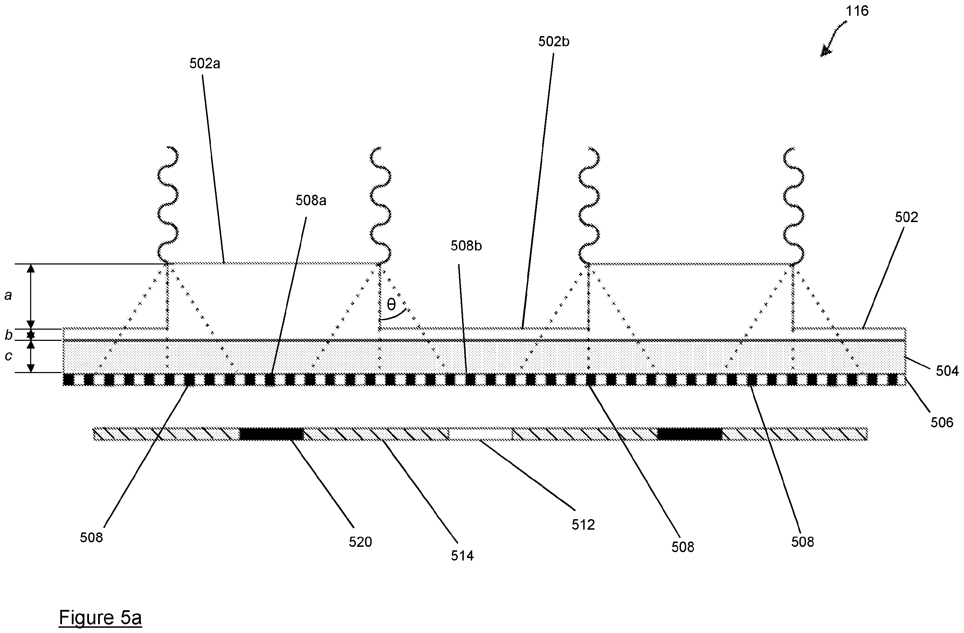

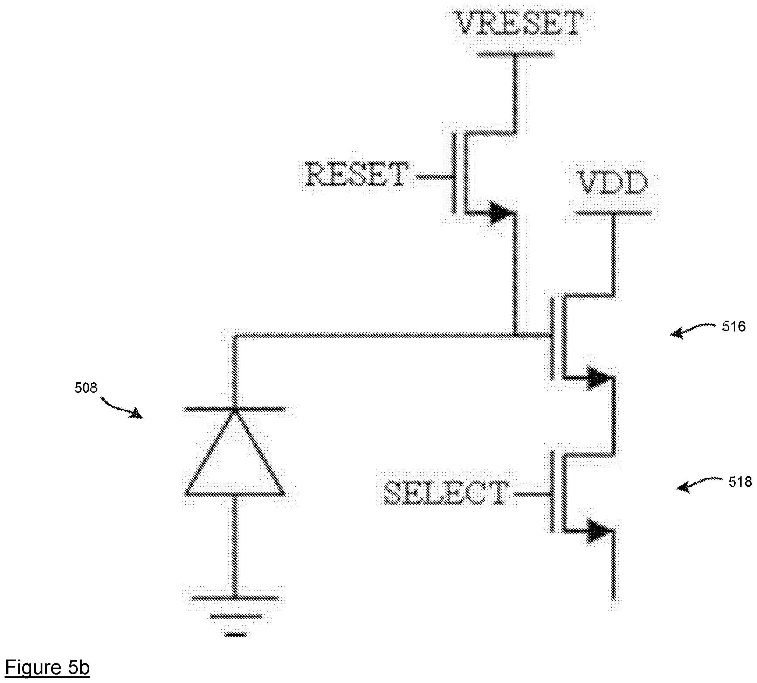

Figure 5a and 5b show the radiation detector 116 in detail, which provides a first embodiment of the present invention. The detector 116 exploits process 408, which has a well-known cross section which allows an accurate measurement of the dose being delivered to the patient.

The detector 116 has a similar construction to the conventional MAPS detector 300 described above with reference to Figures 3 and 4, except that it has a bulk layer which is patterned with a varying thickness, as will be described below.

Referring first to Figure 5a - the detector 116 has first and second silicon layers 502, 504, each arranged to generate charged particles (i.e. electron-hole pairs) in response to incident photons. The first layer 502 is a highly doped (p⁺⁺) bulk layer, like the bulk layer 302 described above, and will be referred to below as the bulk layer 502. The second layer 504 is a grown layer of lightly doped p-type material, like the sensitive layer 304 described above, and will be referred to below as the sensitive layer 504. The sensitive layer 504 is grown on the bulk layer 502 by epitaxy, so these two layers can also be referred to as an epitaxial layer and a substrate layer respectively.

A third layer 506 of silicon is arranged so that the sensitive layer 504 is sandwiched between the bulk layer 502 and the third layer 506. In other words - the sensitive layer 504 is positioned between the layers 502, 504 and in contact with the layers 502, 504.

The third layer 506 comprises a layer of p-type material, throughout which a plurality of collection diodes 508 are positioned, like the electronics layer 306 described above, and will be referred to below as the electronics layer 506. The layers 502, 506 create respective potential barriers trapping the electrons in the sensitive layer 504. Each collection diode 508 forms a potential well to collect the electrons trapped in the sensitive layer 504.

To sum up - the bulk layer 502 is arranged to generate charged particles (such as electrons) in response to photons which are incident on the bulk layer 502, like the bulk layer 302 of the conventional detector of Figure 4. The sensitive layer 504 acts as a signal generation layer (like the sensitive layer 304 of the conventional detector of Figure 4) and is arranged to generate signal charge (such as electrons) in response to charged particles generated in the bulk layer 502 which are incident on the sensitive layer 504. The electronics layer 506 acts as a barrier layer, like the electronics layer 306 of the conventional detector of Figure 4. The sensitive layer 504 is positioned between the bulk layer 502 and electronics layer 506 and in contact with the bulk layer 502 and electronics layer 506. The bulk layer 502 and electronics layer 506 trap the signal charge in the sensitive layer 504. Each collection diode 508 in the electronics layer is arranged to collect the signal charge trapped in the sensitive layer 504, like the diodes 308 in the conventional detector of Figure 4.

Figure 5b shows the in-pixel circuitry of the detector 116 for a single pixel associated with one of the diodes 508. Each pixel has three transistors which are housed in the electronics layer 506 along with the diodes 508. The circuitry comprises a source follower and a select switch 518. The generated electrons are confined by the potential barrier between the lightly doped sensitive layer 504 and the highly doped bulk layer 502. The electrons diffuse through the sensitive layer 504 until they reach the depleted zone above the diode 508. Here the electrons are collected. The amount of collected electrons affect the gate voltage of the source follower 516 and hence affect the current when the select switch 518 is opened.

Unlike the bulk layer 302 in the conventional detector of Figure 4, the bulk layer 502 is patterned with a varying thickness so that it has a plurality of relatively thick regions 502a interspersed with a plurality of relatively thin regions 502b. The thicknesses of the thin regions 502b and the sensitive layer 504 are denoted *b* and c respectively. A thickness *(a+b)* of each of the thick regions 502a is greater than the thickness (b) of each of the thin regions 502b.

The bulk layer 502 is patterned by removing silicon material to form the thin regions 502b. The silicon material may be removed by sawing and etching techniques, for example.

The thickness (*a*+*b*) of each of the thick regions 502a is substantially constant across its entire area, and also the thickness (b) of each of the thin regions 502b is substantially constant across its entire area. Consequently the outer surfaces of the thick regions 502a (the upper surfaces in the orientation of Figure 5a) lie in a first plane substantially perpendicular to the direction of travel of the radiation beam, and the outer surfaces of the thin regions 502b lie in a second plane which is also substantially perpendicular to the direction of travel of the radiation beam. Note that the interface where the sensitive layer 504 contacts the bulk layer 502 is substantially planar.

A few microns (b) of the bulk layer 502 remain across the entire area of the sensor within the thin regions 502b. This maintains the confinement effect of the bulk layer 502 on the electrons as they traverse the sensitive layer 504, so that they can be collected at the collection diodes 508.

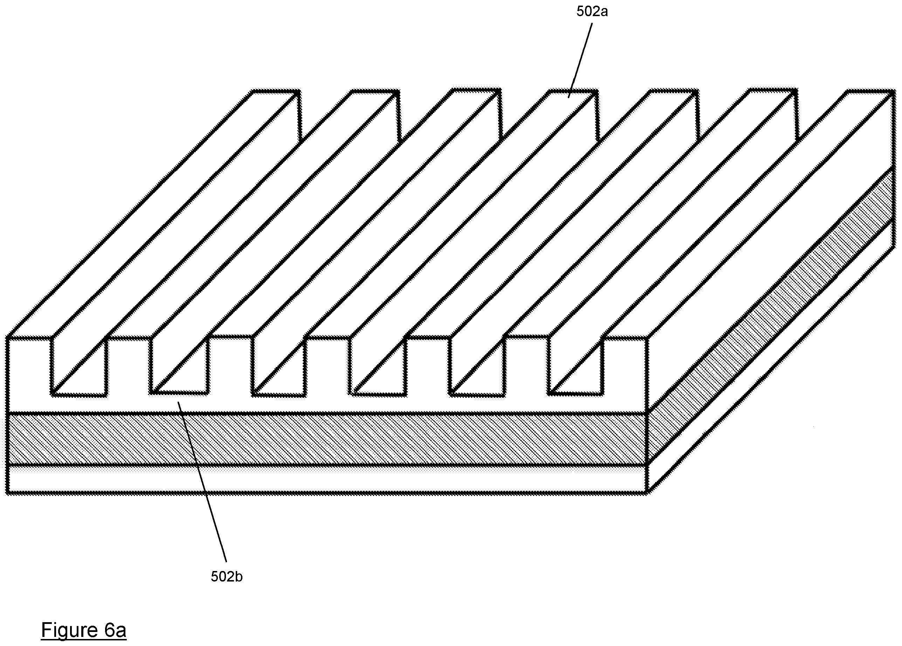

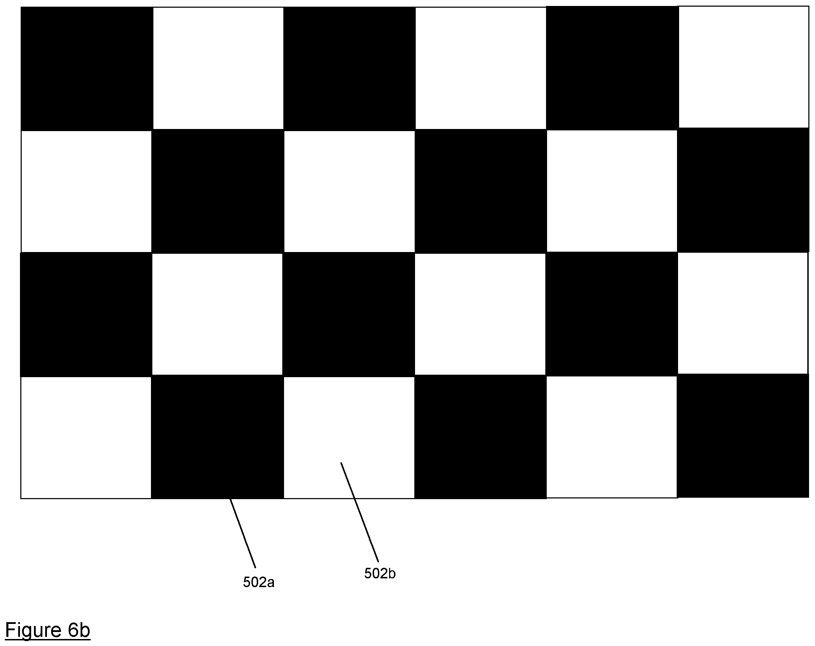

The regions 502a, b may take the form of alternating ridges and trenches that span the surface of the detector, as shown in Figure 6a. Alternatively, the regions 502a, b may take the form of a chequered pattern of pads and recesses, a plan view of which is shown in Figure 6b.

The angle of emission of the electrons produced in a thick region 502a follows a known distribution. For example, for 1 MeV incident photons, the mode scattering angle is 13 degrees to the entrance angle, and approximately 50% of the electrons are scattered within 30 degrees of the entrance angle. This 30 degree mean scattering angle θ is shown in Figure 5a and labelled θ. Due to the large distances travelled by the photons compared to the size of the detector, the entrance angle is approximately perpendicular to the plane of the detector.

As approximately 50% of the electrons are generated within the mean scattering angle θ, and are generated uniformly throughout the layer, it is possible to obtain the collection area where the contribution from the extra electrons produced in the thick regions 502a is maximally uniform. Simple geometry may be used to show that, in the plane of the detector, the maximal uniform generation area has a horizontal distance of (*a*+*b*+*c*)tan*θ* from the edge of the thick regions 502a, directed towards the centre of the thick regions 502a. This maximal collection area where the contribution from the extra electrons is uniformly maximal is indicated by black portions 520 of a scale bar accompanying Figure 5a.

Similarly, it is possible to determine the collection area where the signal due to the extra electrons produced in the thick region 502a is minimal. It may again be shown through simple geometry that the uniformly minimal generation area has a horizontal distance of (*a*+*b*+*c*)tan*θ* from the edge of the thick regions 502a, directed towards the centre of the thin regions 502b. This minimal collection area where the contribution from the extra electrons is uniformly minimal is indicated by a white portion 512 of the scale bar accompanying Figure 5a.

Each transition region, in which the signal due to extra electrons produced in the raised portions 504 varies, spans a horizontal distance of 2(*a*+*b*+*c*)tan*θ*. These are indicated by hashed portions 514 of the scale bar accompanying Figure 5a. In one embodiment, this intermediate transition signal may be exploited for analysis.

In order to measure the signal in the areas 520 and 512, a minimum of one diode is required to exist completely within the area in question. Preferably, a minimum of three diodes exist completely within the area in question. The pixel pitch may, for example, be 14*µ*m. This pixel pitch may, for example, be larger if only one diode existed completely within the area in question. In the embodiment of Figure 5a there are two or three diodes 508a within each maximal collection area 520, and two or three diodes 508b within each minimal collection area 512.

Once the signals in the areas 520 and 512 have been measured, it is then possible to subtract the signal from the diodes 508b in the minimal collection area 512 from the signal from the diodes 508a in the maximal collection area 520 to obtain the signal originating exclusively from process 408 in the thick regions 502a of the sensor, through the thickness (*a*). This signal will be substantially free from background contamination.

More specifically, consider a first diode 508b in the minimal collection area 512 adjacent a first one 502b of the thin regions and a second diode 508a in the maximal collection area 520 adjacent a first one 502a of the thick regions which is a neighbour of the first one 502b of the thin regions. The analysis unit 122 obtains a first signal from the first diode 508b and a second signal from the second diode 508a, and subtracts the first signal from the second signal to obtain an output signal originating exclusively from electrons generated in the first one 502a of the thick regions.

Process 408 has a well-known cross-section with respect to the entire radiation beam, and it is thus possible to determine the signal of the entire radiation beam in the thick region 502a.

The first and second signals mentioned above may represent the rate of electrons as they are collected at the collection diodes 508a, b. In this case, it is then possible to integrate this rate over the total time period that the radiation beam has been turned on to obtain the total dose delivered in that time.

The pitch of the modulation structure (i.e. the distance from the edge of one thick region to the same edge on an adjacent thick region) will be equal to 4(*a*+*b*+*c*)tan*θ* + N, where N is the total combined number of pixels used in both the maximal 520 and minimal 512 collection areas. The thickness c of the sensitive layer 504 may be approximately 1-50*µ*m. The thickness b of the remaining bulk material in the thin region 502b is only required to be a few microns. The thickness *a* is required to be a few times that of the thickness *b,* whilst also being of about the same order as the thickness c of the sensitive layer 504, so as to allow the extra signal to be of the same order as the signal generated in the sensitive layer 504. Therefore, the thickness *a* may be approximately 20*µ*m, Accordingly, taking N=6, the modulation structure pitch may be approximately 180*µ*m, and the width of a single thick or thin region may therefore be approximately 90*µ*m.

The thickness of the radiation detector upstream must be relatively low, so that it does not interfere unduly with the beam directed towards the patient. Preferably the thickness (a+b) of each of the thick regions 502a of the patterned structure is less than 200*µ*m, and most preferably is it less than 100*µ*m. By way of example the thickness (a+b) may be of the order of 20*µ*m. In Figure 5a the thickness (a+b) is constant, but if it varies then the maximum or average thickness is less than 100*µ*m or 200*µ*m.

The thickness (b) of each of the thin regions 502b of the patterned structure is non-zero - typically it is greater than 1*µ*m. In Figure 5a the thickness b is constant, but if it varies then the minimum or average thickness is greater than 1*µ*m.

The thickness (b) of each of the thin regions 502b of the patterned structure is typically less than 20*µ*m and more preferably it is less than 10*µ*m. - for example it may be between 1*µ*m and 5*µ*m.

A sum of the thicknesses of the patterned structure (thickness a+b), the electronics layer 506, and the signal generation layer (thickness c) has a maximum which is typically less than 500*µ*m, preferably less than 300*µ*m and most preferably less than 200*µ*m. In this case the radiation detector has no more layers, so this sum gives the total thickness of the radiation detector.

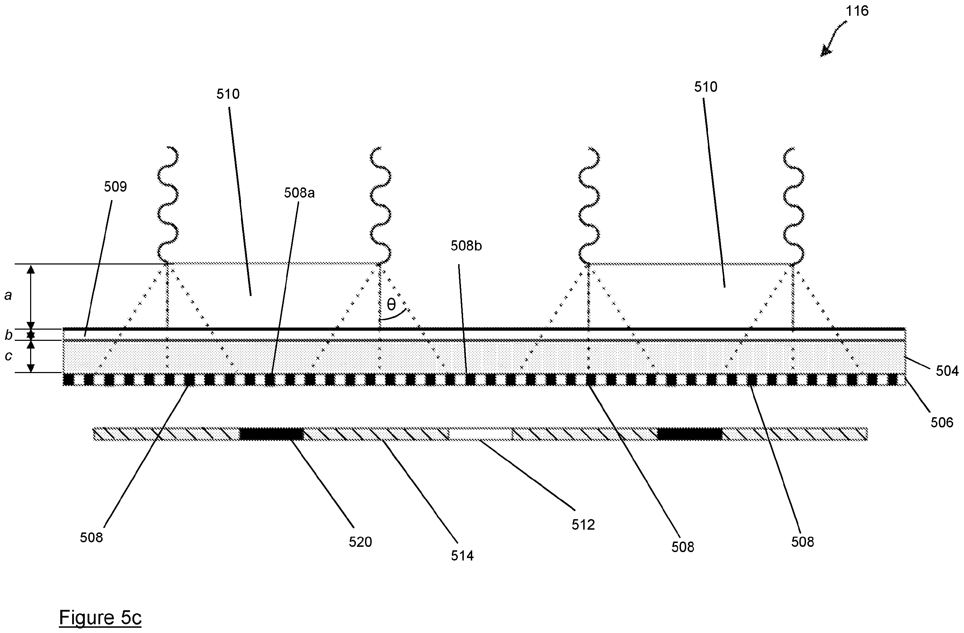

The bulk layer 502 provides a patterned structure, which is patterned with a varying thickness so that it has a plurality of relatively thick regions 502a interspersed with a plurality of relatively thin regions 502b, a thickness of each of the thick regions 502a being greater than a thickness of each of the thin regions 502b. In this example the thin and thick regions together form a single layer of material. In an alternative embodiment of the invention, shown in Figure 5c, the patterned structure is instead formed by a thin layer 509 carrying strips 510 of a different material. The thin layer 509 is in contact with the sensitive layer 504 and provides the barrier trapping the electrons in the sensitive layer 504. The strips 510 provide the thick regions of the patterned structure, and the thin layer 509 provides the thin regions of the patterned structure between the strips 510. The strips 520 and the thin layer 509 may be formed from different materials. For instance the thin layer 509 may be made of foil and the strips may be made of steel or tungsten.

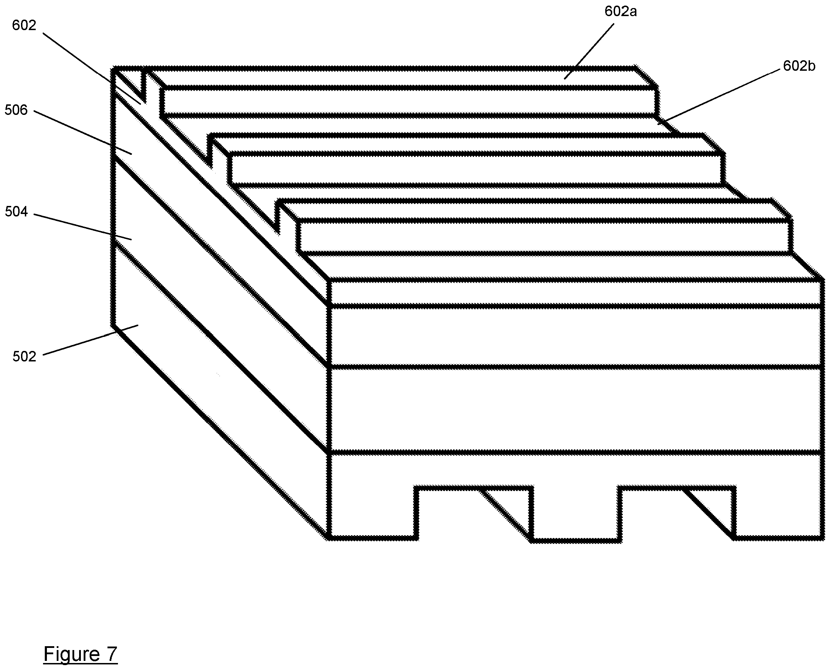

Figure 7 shows an alternative radiation detector which is similar to the detector 116 described above with reference to Figures 5a and 6a, and the same reference numbers are used for equivalent features. Note that the detector of Figure 7 is depicted upside down relative to the detector 116 of Figure 5a.

A further patterned structure 602 is arranged to generate charged particles (such as electrons and positrons) in response to incident photons. The further patterned structure 602 is positioned at the back of the detector, so that the electronics layer 506 is positioned between the sensitive layer 504 and the further patterned structure 602. The further patterned structure is patterned with a varying thickness in a similar fashion to the bulk layer 502, so that it has a plurality of thick regions 602a interspersed with a plurality of thin regions 602b, a thickness of each of the thick regions being greater than a thickness of each of the thin regions.

The further patterned structure 602 may be Silicon Oxide, or it may be incorporated in the housing of the detector.

The regions 602a, b form a series of alternating trenches and ridges, running at right angles to the trenches and ridges in the bulk layer 502. Alternatively, they could be running at an oblique angle to the trenches and ridges in the bulk layer 502. This enables the analysis unit 22 to disentangle the amount of radiation coming from either side of the detector.

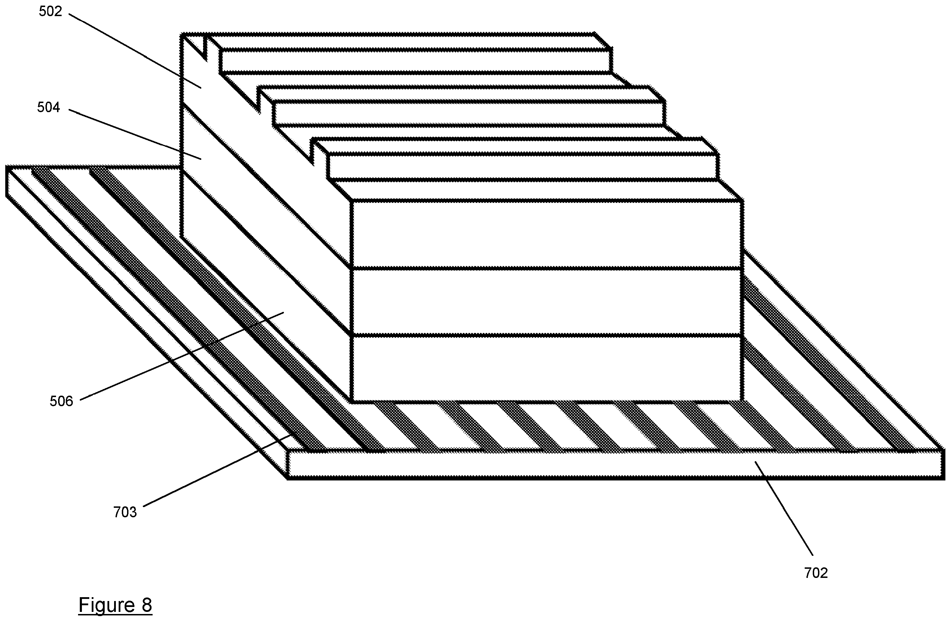

Figure 8 shows a further alternative embodiment. In this case, a housing 702 of the detector carries wires 703 which run at right angles to the trenches and ridges in the bulk layer 502. Alternatively, they could be running at an oblique angle to the trenches and ridges in the bulk layer 502. The housing 702 comprises a carbon foam or a SiC foam, and the wires 703 can be made from any material that results in a different rate of scattering. The housing 702 and wires 703 form a patterned structure which enables the analysis unit 22 to disentangle the amount of radiation coming from either side of the detector.

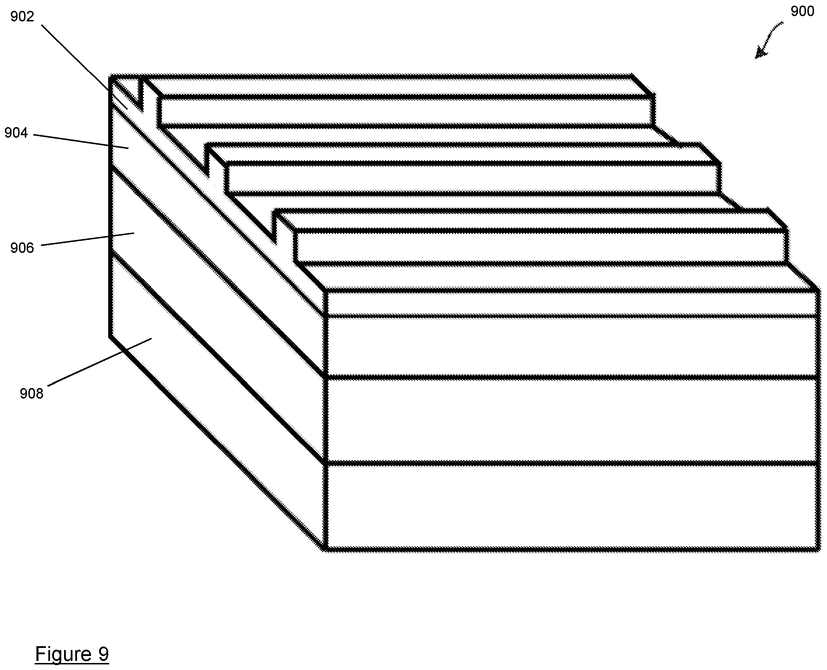

Although the embodiments described above use a MAPS detector, it will be appreciated that the invention could equally be implemented in other types of detectors comprising a sensitive layer, at least one collector, and means for confining charges in the sensitive layer. Such means may comprise doping differences between the sensitive layer and any adjacent layers, as has been described above, or it may alternatively comprise another means for generating an electric field to confine the charges, such as a series of metallic contacts and wires. An example of an alternate detector that may be used is a 3D sensor, and such an embodiment is shown in Figure 9.

The 3D sensor, shown generally at 900, comprises a patterned bulk layer 902, a sensitive layer 904 adjacent to the bulk layer 902, an analogue electronics layer 906 adjacent to the sensitive layer 904 which includes a series of collectors at the interface between the two layers, and a digital circuitry layer 908 adjacent to the analogue electronics layer 906.

Although the invention has been described above with reference to one or more preferred embodiments, it will be appreciated that various changes or modifications may be made without departing from the scope of the invention as described in the appended claims.

Further, although the invention has been described with reference to an upstream detector for use in radiotherapy, it will be appreciated that the principles hereinbefore described could equally be applied to numerous other fields, including nuclear and dosimetry applications when mixed radioactive sources are used.

## Claims

1. A radiation detector (116) comprising: a patterned structure (502) arranged to generate charged particles in response to photons which are incident on the patterned structure, a signal generation layer (504) arranged to generate signal charge in response to charged particles generated in the patterned structure which are incident on the signal generation layer; a barrier layer (506) arranged so that the signal generation layer is positioned between the patterned structure and barrier layer and in contact with the patterned structure and barrier layer, wherein the patterned structure and barrier layer trap the signal charge in the signal generation layer; and a plurality of collectors (508), each collector arranged to collect the signal charge trapped in the signal generation layer,
wherein the patterned structure is patterned with a varying thickness so that it has a plurality of thick regions (502a) interspersed with a plurality of thin regions (502b), a thickness of each of the thick regions being greater than a thickness of each of the thin regions.

2. The radiation detector of claim 1, wherein a total number of the collectors is greater than a sum of the number of thick regions and the number of thin regions.

3. The radiation detector of any preceding claim, wherein the total number of collectors is more than four times greater than the sum of the number of thick regions and the number of thin regions.

4. The radiation detector of any preceding claim, wherein a maximum or average thickness of each of the thick regions of the patterned structure is less than 200*µ*m.

5. The radiation detector of any preceding claim, wherein a minimum or average thickness of each of the thin regions of the patterned structure is greater than 1*µ*m.

6. The radiation detector of any preceding claim, further comprising a further patterned structure arranged to generate charged particles in response to photons which are incident on the further patterned structure, wherein the barrier layer is positioned between the signal generation layer and the further patterned structure, and the further patterned structure is patterned with a varying thickness so that it has a plurality of thick regions interspersed with a plurality of thin regions, a thickness of each of the thick regions of the further patterned structure being greater than a thickness of each of the thin regions of the further patterned structure.

7. The radiation detector of any preceding claim, further comprising a plurality of wires arranged to generate charged particles in response to photons which are incident on the plurality of wires, wherein the barrier layer is positioned between the signal generation layer and the plurality of wires.

8. The radiation detector of any preceding claim, wherein the radiation detector is a monolithic active pixel sensor.

9. The radiation detector of any preceding claim, wherein the patterned structure contacts the signal generation layer at an interface which is substantially planar.

10. The radiation detector of any preceding claim, wherein the patterned structure, signal generation layer and barrier layer are respectively first, second and third semiconductor layers.

11. The radiation detector of claim 10, wherein the first, second and third semiconductor layers have different doping concentrations, and the different doping concentrations create potential barriers trapping the signal charge in the signal generation layer.

12. A radiation detection system comprising:
the radiation detector of any preceding claim, wherein the collectors comprise a first collector adjacent a first one of the thin regions and a second collector adjacent a first one of the thick regions; and
an analysis unit coupled to the radiation detector, wherein the analysis unit is arranged to: obtain a first signal from the first collector; obtain a second signal from the second collector; and correct the second signal using the first signal to obtain an output.

13. A radiotherapy system comprising: a radiation beam source to generate a radiation beam; and a radiation detector according to any of claims 1 to 11 arranged to detect photons from the radiation beam.

14. A method of operating the radiation detector (116) of any of claims 1 to 11, wherein the collectors comprise a first collector (508b) adjacent a first one of the thin regions (502b) and a second collector (508a) adjacent a first one of the thick regions (502a), and the method comprises:
obtaining a first signal from the first collector;
obtaining a second signal from the second collector; and
correcting the second signal using the first signal to obtain an output.

15. A method of manufacturing the radiation detector (116) of any of claims 1 to 11, the method comprising removing material from the patterned structure (502) to form the thin regions (502b).

## Patentansprüche

1. Strahlungsdetektor (116), umfassend: eine gemusterte Struktur (502), die angeordnet ist zum Erzeugen von geladenen Teilchen in Reaktion auf Photonen, die auf der gemusterten Struktur einfallen, eine Signalerzeugungsschicht (504), die angeordnet ist zum Erzeugen einer Signalladung in Reaktion auf in der gemusterten Struktur erzeugte geladene Teilchen, die auf der Signalerzeugungsschicht einfallen; eine Sperrschicht (506), die derart angeordnet ist, dass die Signalerzeugungsschicht zwischen der gemusterten Struktur und der Sperrschicht positioniert ist und sich mit der gemusterten Struktur und der Sperrschicht in Kontakt befindet, wobei die gemusterte Struktur und die Sperrschicht die Signalladung in der Signalerzeugungsschicht einfangen; und eine Mehrzahl von Kollektoren (508), wobei jeder Kollektor angeordnet ist zum Sammeln der in der Signalerzeugungsschicht eingefangenen Signalladung,
wobei die gemusterte Struktur mit variierender Dicke gemustert ist, so dass sie eine Mehrzahl von dicken Bereichen (502a) aufweist, die mit einer Mehrzahl von dünnen Bereichen (502b) durchsetzt sind, wobei eine Dicke jedes der dicken Bereiche größer ist als eine Dicke jedes der dünnen Bereiche.

2. Strahlungsdetektor nach Anspruch 1, wobei eine Gesamtzahl der Kollektoren größer ist als eine Summe der Anzahl von dicken Bereichen und der Anzahl von dünnen Bereichen.

3. Strahlungsdetektor nach einem der vorangehenden Ansprüche, wobei die Gesamtzahl der Kollektoren mehr als viermal größer ist als die Summe der Anzahl von dicken Bereichen und der Anzahl von dünnen Bereichen.

4. Strahlungsdetektor nach einem der vorangehenden Ansprüche, wobei eine maximale oder eine durchschnittliche Dicke jedes der dicken Bereiche der gemusterten Struktur weniger als 200 µm beträgt.

5. Strahlungsdetektor nach einem der vorangehenden Ansprüche, wobei eine minimale oder eine durchschnittliche Dicke jedes der dünnen Bereiche der gemusterten Struktur mehr als 1 µm beträgt.

6. Strahlungsdetektor nach einem der vorangehenden Ansprüche, ferner umfassend eine weitere gemusterte Struktur, die angeordnet ist zum Erzeugen von geladenen Teilchen in Reaktion auf Photonen, die auf der weiteren gemusterten Struktur einfallen, wobei die Sperrschicht zwischen der Signalerzeugungsschicht und der weiteren gemusterten Struktur positioniert ist und die weitere gemusterte Struktur mit variierender Dicke gemustert ist, so dass diese eine Mehrzahl von dicken Bereichen aufweist, die mit einer Mehrzahl von dünnen Bereich durchsetzt sind, wobei eine Dicke jedes der dicken Bereiche der weiteren gemusterten Struktur größer ist als eine Dicke jedes der dünnen Bereiche der weiteren gemusterten Struktur.

7. Strahlungsdetektor nach einem der vorangehenden Ansprüche, ferner umfassend eine Mehrzahl von Drähten, die angeordnet sind zum Erzeugen von geladenen Teilchen in Reaktion auf Photonen, die auf den mehrzähligen Drähten einfallen, wobei die Sperrschicht zwischen der Signalerzeugungsschicht und den mehrzähligen Drähten positioniert ist.

8. Strahlungsdetektor nach einem der vorangehenden Ansprüche, wobei der Strahlungsdetektor ein monolithischer aktiver Pixelsensor ist.

9. Strahlungsdetektor nach einem der vorangehenden Ansprüche, wobei die gemusterte Struktur die Signalerzeugungsschicht an einer Schnittstelle kontaktiert, die im Wesentlichen eben ist.

10. Strahlungsdetektor nach einem der vorangehenden Ansprüche, wobei die gemusterte Struktur, die Signalerzeugungsschicht und die Sperrschicht jeweils eine erste, zweite und dritte Halbleiterschicht sind.

11. Strahlungsdetektor nach Anspruch 10, wobei die erste, zweite und dritte Halbleiterschicht jeweils verschiedene Dotierungskonzentrationen aufweisen und die verschiedenen Dotierungskonzentrationen potenzielle Barrieren bilden, die die Signalladung in der Signalerzeugungsschicht abfangen.

12. Strahlungsdetektionssystem, umfassend:
den Strahlungsdetektor nach einem der vorangehenden Ansprüche, wobei die Kollektoren einen ersten Kollektor umfassen, der einem ersten der dünnen Bereiche benachbart ist, und einen zweiten Kollektor, der einem ersten der dicken Bereiche benachbart ist; und
eine mit dem Strahlungsdetektor gekoppelte Analyseeinheit, wobei die Analyseeinheit angeordnet ist zum Beziehen eines ersten Signals von dem ersten Kollektor; zum Beziehen eines zweiten Signals von dem zweiten Kollektor; und zum Korrigieren des zweiten Signals anhand des ersten Signals, um eine Ausgabe zu erhalten.

13. Strahlentherapiesystem, umfassend: eine Strahlquelle zum Erzeugen eines Strahls; und einen Strahlungsdetektor nach einem der Ansprüche 1 bis 11, der angeordnet ist zum Detektieren von Photonen aus dem Strahl.

14. Verfahren zum Betreiben des Strahlungsdetektors (116) nach einem der Ansprüche 1 bis 11, wobei die Kollektoren einen ersten Kollektor (508b) umfassen, der einem ersten der dünnen Bereiche (502b) benachbart ist, und einen zweiten Kollektor (508a), der einem ersten der dicken Bereiche (502a) benachbart ist, wobei das Verfahren umfasst:
das Beziehen eines ersten Signals von dem ersten Kollektor;
das Beziehen eines zweiten Signals von dem zweiten Kollektor; und
das Korrigieren des zweiten Signals anhand des ersten Signals, um eine Ausgabe zu erhalten.

15. Verfahren zum Herstellen des Strahlungsdetektors (116) nach einem der Ansprüche 1 bis 11, wobei das Verfahren das Abtragen von Material von der gemusterten Struktur (502) umfasst, um die dünnen Bereiche (502b) zu bilden.

## Revendications

1. Détecteur de radiations (116) comprenant une structure à motifs (502) organisée pour générer des particules chargées en réponse aux photons incidents dans la structure à motifs, une couche de génération de signal (504) organisée pour générer une charge de signal en fonction des particules chargées générées dans la structure à motifs, qui arrivent sur la couche de génération de signal ; une couche-barrière (506) organisée de façon que la couche de génération de signal soit placée entre la structure à motifs et la couche barrière et contact avec la structure à motifs et la couche-barrière, la structure à motifs et la couche barrière piégeant la charge de signal de la couche de génération de signal ainsi qu'un ensemble de collecteurs (508), chaque collecteur recevant la charge de signal piégée dans la couche de génération de signal,
la structure à motifs ayant des motifs d'épaisseur variable de façon à avoir un ensemble de régions épaisses (502a) intercalées avec un ensemble de régions minces (502b), l'épaisseur de chacune des régions épaisses étant supérieure à l'épaisseur de chacune des régions minces.

2. Détecteur de radiations selon la revendication 1,
selon lequel
le nombre total de collecteurs est supérieur à la somme du nombre de régions épaisses et du nombre de régions minces.

3. Détecteur de radiations selon l'une quelconque des revendications précédentes,
selon lequel
le nombre total de collecteurs est plus de quatre fois supérieur à la somme du nombre de régions épaisses et du nombre de régions minces.

4. Détecteur de radiations selon l'une quelconque des revendications précédentes,
selon lequel
l'épaisseur maximale ou moyenne de chacune des régions épaisses de la structure à motifs est inférieure à 200 µm.

5. Détecteur de radiations selon l'une quelconque des revendications précédentes,
selon lequel
l'épaisseur minimale ou moyenne de chacune des régions minces de la structure à motifs est supérieure à 1 µm.

6. Détecteur de radiations selon l'une quelconque des revendications précédentes,
comprenant en outre une autre structure à motifs organisée pour générer des particules chargées en réponse aux photons incidents dans l'autre structure à motifs,
la couche barrière étant placée entre la couche de génération de signal et l'autre structure à motifs et cette autre structure à motifs ayant des motifs d'épaisseur variable de façon à avoir un ensemble de régions épaisses intercalées entre un ensemble de régions minces, l'épaisseur de chacune des régions épaisses de cette autre structure à motifs étant supérieure à l'épaisseur de chacune des régions minces de l'autre structure à motifs.

7. Détecteur de radiations selon l'une quelconque des revendications précédentes,
comprenant en outre
un ensemble de câbles organisés pour générer des particules chargées en réponse aux photons incidents dans l'ensemble des câbles,
la couche-barrière étant placée entre la couche de génération de signal et l'ensemble des câbles.

8. Détecteur de radiations selon l'une quelconque des revendications précédentes,
selon lequel
le détecteur de radiation est un capteur de pixels monolithique, actif.

9. Détecteur de radiations selon l'une quelconque des revendications précédentes,
selon lequel
la structure à motifs touche la couche de génération de signal à une interface qui est pratiquement plane.

10. Détecteur de radiations selon l'une quelconque des revendications précédentes,
selon lequel
la structure à motifs, la couche de génération de signal et la couche barrière sont respectivement une première, une seconde et une troisième couche semi-conductrice.

11. Détecteur de radiations selon la revendication 10,
selon lequel
la première, la seconde et la troisième couches semi-conductrices ont des concentrations de dopage différentes et ces différentes concentrations de dopage créent des barrières de potentiel piégeant la charge de signal dans la couche de génération de signal.

12. Système de détection de radiations comprenant :
- le détecteur de radiations selon l'une quelconque des revendications précédentes, les collecteurs ayant un premier collecteur adjacent à la première des régions minces et un second collecteur adjacent à la première des régions épaisses et,
- une unité d'analyse couplée au détecteur de radiations, cette unité d'analyse étant organisée pour obtenir un premier signal du premier collecteur, un second signal du second collecteur et corriger le second signal en utilisant le premier signal pour obtenir une sortie.

13. Système de radiothérapie comprenant :
une source de faisceaux de radiations pour générer un faisceau de radiations et un détecteur de radiations selon l'une quelconque des revendications 1 à 11 organisé pour détecter les photons émis par le faisceau de radiations.

14. Procédé de fonctionnement d'un détecteur de radiation (116) selon l'une quelconque des revendications 1 à 11,
selon lequel
les collecteurs ont un premier collecteur (508b) adjacent à une première région des régions minces (502b) et un second collecteur (508a) adjacent à une première région des régions épaisses (502a),
le procédé consistant à :
- obtenir un premier signal du premier collecteur,
- obtenir un second signal du second collecteur et,
- corriger le second signal en utilisant le premier signal pour obtenir une sortie.

15. Procédé de fabrication d'un détecteur de radiations (116) selon l'une quelconque des revendications 1 à 11,
procédé consistant à
enlever la matière de la structure à motifs (502) pour former les régions minces (502b).
